(19) Europäisches Patentamt
European Patent Office
Office européen des brevets

(11) Veröffentlichungsnummer: **0 003 749**
**B1**

(12) # EUROPÄISCHE PATENTSCHRIFT

(45) Veröffentlichungstag der Patentschrift: **15.09.82**

(51) Int. Cl.³: **A 01 N 43/70,** C 07 D 251/52, C 09 D 5/16

(21) Anmeldenummer: **79100173.8**

(22) Anmeldetag: **22.01.79**

(54) **2-Methylthio-4-cyclopropylamino-6-(Alpha,Beta-dimethylpropylamino)-s-triazin und die Verwendung von Triazinderivaten als Meerwasseralgizide.**

(30) Priorität: **03.02.78 CH 1212/78**

(43) Veröffentlichungstag der Anmeldung: **05.09.79 Patentblatt 79/18**

(45) Bekanntmachung des Hinweises auf die Patenterteilung: **15.09.82 Patentblatt 82/37**

(84) Benannte Vertragsstaaten: **DE FR IT NL SE**

(56) Entgegenhaltungen:
**DE-A-1 914 014**
**FR-A-1 565 231**
**US-A-4 012 503**

(73) Patentinhaber: **CIBA-GEIGY AG, Patentabteilung Postfach, CH-4002 Basel (CH)**

(72) Erfinder: **Berrer, Dagmar, Dr., Steingrubenweg 92, CH-4125 Riehen (DE)**
Erfinder: **Lorenz, Joachim, Dr., Im Tiefen Weg 17, D-6140 Bensheim 3 (DE)**
Erfinder: **Grade, Reinhardt, Dr., Waldstrasse 22, D-6144 Zwingenberg-Rodau (DE)**

**EP 0 003 749 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 2-Methylthio-4-cyclopropylamino-6-(α,β-dimethylpropylamino)-s-triazin und die Verwendung von Triazinderivaten als Meerwasseralgizide

Die vorliegende Erfindung betrifft die Verwendung von Triazinen als Meerwasseralgizide sowie 2-Methylthio-4-cyclopropylamino-6-(α,β-dimethylpropylamino)-s-triazin und dessen Verwendung als Algizid.

Es ist z. B. aus der DE-A-1 914 014 bekannt, 2-Alkylthio-4,6-diamino-s-triazin als selektive Mittel zur Bekämpfung von Unkräutern und Ungräsern einzusetzen.

In FR-A-1 565 231 werden herbizide Mittel, die 2-Äthylamino-4-(α,β-dimethylpropylamino)-6-methyl-mercapto-1,3,5-triazin enthalten, beansprucht. Über die Eignung dieser Substanzen als Meerwasseralgizid wird keine Angabe gemacht.

In neuerer Zeit erkannte man auch die gute Wirksamkeit solcher Triazine gegen Süßwasseralgen. So ist beispielsweise in der JP-A-49 061-336 ihre Verwendung als Algizide gegen Süßwasseralgen wie Spirogyra, Hydrodiction und Oedogeniales beschrieben worden. In der JP-A-52 021-331 und dem US Pat. Nr. 4 012 503 wird berichtet, daß verwandte Triazine auch eine gewisse Aktivität gegen Meerwasseralgen entwickeln. Trotz niedrigen minimalen Hemmkonzentrationen (MIC) sind die erwähnten Triazine gegen Meerwasseralgen nur unzureichend wirksam, da sie ihre volle Aktivität (welche in den niedrigen MIC-Werten zum Ausdruck kommt) erst nach langer Einwirkungszeit entwickeln. Algen brauchen eine bestimmte Dauer ruhendes Wasser, um sich richtig an zu schützenden Oberflächen, wie Schiffskörper etc. festsetzen zu können. Es hat sich gezeigt, daß eine Zeit von 2 bis 4 Stunden für Meerwasseralgen ausreichend ist, um so fest anzuwachsen, daß sie nicht durch Turbulenz weggespült werden. Von einem Meerwasseralgizid wird daher gefordert, daß es sehr rasch eine hohe Wirksamkeit entfaltet.

Es wurde nun überraschend eine kleine Gruppe von Triazinen gefunden, welche, verglichen mit den bekannten Triazin-Algiziden, ihre Wirksamkeit bei vergleichbaren MIC-Werten rascher entwickeln, was ihnen erst große praktische Bedeutung verleiht.

Die vorliegende Erfindung betrifft daher die Verwendung von 2-Methylthio-4-cyclopropylamino-6-(α,β-dimethylpropylamino)-s-triazin (I), 2-Methylthio-4-cyclopropylamino-6-t.butylamino-s-triazin (II) und 2-Methylthio-4-äthylamino-6-(α,β-dimethylpropylamino)-s-triazin (III) als Algizide gegen Meerwasseralgen. Die Triazine (II) und (III) sind als Herbizide bekannte Verbindungen, Triazin (I) ist eine neue Verbindung.

Die Herstellung der erfindungsgemäß zu verwendenden Verbindungen erfolgt auf bekannte Weise und ist in der DE-A-1 914 014 beschrieben.

Es kann dabei Cyanurchlorid mit einem Amin ausgewählt aus der Gruppe Cyclopropylamin, t.Butylamin, Äthylamin oder α,β-Dimethylpropylamin umgesetzt werden und das als Zwischenprodukt erhaltene Dichloramino-s-triazin mit Methylmercaptan bzw. einem Salzes davon in ein Methylthio-chloramino-s-triazin-Derivat überführt werden, das dann mit dem in der ersten Stufe nicht verwendeten Amin umgesetzt wird, um den zweiten in den Formel I, II und III erwähnten Amin-Substituenten einzuführen. Es ist auch möglich die Reihenfolge der 2. und 3. Stufe des oben beschriebenen Reaktionsverlaufs auszutauschen. Die Methylthiogruppe wird in diesem Falle wie in der DE-A-1 914 014 beschrieben mittels Thioharnstoff und Dimethylsulfat in Gegenwart basischer Stoffe eingeführt. Bevorzugt wird in allen Reaktionsschritten ein säurebindendes Mittel, wie ein tertiäres Amin, ein Alkalimetall-Hydroxid oder -alkoholat eingesetzt, und die Reaktionen werden in einem inerten organischen Lösungsmittel durchgeführt. Dabei ist die Verwendung von Ketonen, wie Aceton oder Methyläthylketon besonders geeignet.

Die erfindungsgemäßen Verbindungen gelangen überall dort zur Anwendung, wo Objekte, welche gegen den Bewuchs von Algen geschützt werden sollen, dem Meerwasser ausgesetzt sind. Es handelt sich dabei insbesondere um Schiffskörper, Wasserbauten, Bojen oder Fischernetze, jedoch auch Kühl- bzw. Rohrsysteme, welche von Meerwasser durchflossen werden. Allgemein schützen die erwähnten Triazine alle Materialien, welche mit Meerwasser in Kontakt treten, vor Algenbewuchs, z. B. Holz, Zellstoff, Textilien und Leder, Farben, Lacke, z. B. Antifoulingfärben und ähnlichen Beschichtungsstoffe, optische und andere Gläser, Kunststoffe, Gummi und Klebestoffe, sowie andere Materialien.

Die Verbindungen werden je nach Verwendungszweck in den dem Fachmann bekannten Konzentrationsbereichen eingesetzt. Die Grenzen der gebräuchlichen Konzentrationen sind durch folgende Werte gegeben: Während in Kühlwasser bereits Konzentrationen im ppm-Bereich genügen, so sind in Antifoulingrezepturen Konzentrationen bis 40 Gew.-% üblich.

Die Verbindungen können in reiner Form oder zusammen mit Trägerstoffen als Stäube-, Streu- oder Nebelmittel appliziert werden. Sie können auch in flüssigen Medien gelöst oder suspendiert werden, wobei gegebenenfalls zur Bildung von gleichmässigen Dispersionen, Netzmittel oder Emulgiermittel die gleichmäßige Verteilung des Wirkstoffs fördern kann. Es können bevorzugt weitere Biocide hinzugefügt werden.

Ein besonders bevorzugter Anwendungsbereich sind Schutzanstrichmittel, insbesondere Antifoulingfarben, die neben den üblichen Grund- und Zusatzstoffen 0,5–40 Gew.-%, vorzugsweise 3 bis 15 Gew.-%, bezogen auf die Gesamtmischung, einer Verbindung der Formeln I, II oder III oder deren Gemische enthalten.

Übliche Grundstoffe für Antifoulingfarben sind die als Bindemittel bezeichneten und dem Fachmann bekannten Lackrohstoffe wie natürliche und synthetische Harze, homo- und copolymere Produkte mit den Monomeren Vinylchlorid, Vinylidenchlorid, Styrol, Vinyltoluol, Vinylestern, Acrylsäure und Methacrylsäure sowie deren Estern, ferner Chlorkautschuk, natürlicher und synthetischer Kautschuk, gegebenenfalls chloriert oder cyclisiert, auch Reaktionsharze wie Epoxidharze, Polyurethanharze, ungesättigte Polyester die gegebenenfalls durch Zusatz von Härtern in filmbildende höhermolekulare Produkte überführt werden können.

Die Bindemittel können flüssig sein oder in gelöster Form vorliegen. Bei gelösten Bindemitteln, auch Thermoplasten, kann ein Schutzfilm auch durch Verdampfen des Lösungsmittels gebildet werden. Feste Beschichtungsmittel können z. B. im Pulverbeschichtungsverfahren auf Gegenstände aufgebracht werden. Weitere übliche Grundstoffe sind z. B. Teer, Modifikatoren, Farbstoffe, anorganische oder organische Pigmente, Füllstoffe und Härter.

Die erfindungsgemäßen Verbindungen können schließlich auch in elastomeren Beschichtungen Anwendung finden, oder auch in Kunststoffe eingearbeitet werden.

In der Praxis werden algizid wirkende Substanzen vielfach mit anderen Biociden eingesetzt. Als vorteilhaft erweist sich im vorliegenden Fall die Kombination mit einem Biocid, das gegen tierische Bewuchsorganismen wirkt. Dabei kommen z. B. $Cu_2O$, Zinkoxid, Triorganozinnverbindungen, wie Tributylzinnoxid, Tributylzinnfluorid oder Triphenylzinnchlorid in Frage, oder allgemein solche Stoffe, die dem Fachmann als gegen tierischen Bewuchs wirksam bekannt sind. In vielen Fällen ist auch die Kombination mit weiteren Algiziden von Vorteil.

Die Erfindung betrifft daher auch die ein Triazin I, II oder III enthaltenden Stoffzusammensetzungen. Vorzugsweise handelt es sich dabei um ein Schutzanstrichmittel und insbesondere um eine Antifoulingfarbe enthaltend ein Triazin I, II oder III.

Die Triazine I, II und III sind wirksame Algizide gegen in Meerwasser auftretende Algenarten. Als wichtigste und häufigste Art sei hier die Enteromorpha erwähnt.

Im weiteren betrifft die vorliegende Erfindung das Triazin I, 2-Methylthio-4-cyclopropylamino-6-($\alpha,\beta$-dimethylpropylamino)-s-triazin, welches eine neue Verbindung ist. Die Verbindung besitzt neben den erwähnten guten Eigenschaften zur Bekämpfung von Meeresalgen auch Wirksamkeit gegen Süßwasseralgen, was ihre Anwendung in diversen Wassersystemen, wie Kühlwasseranlagen oder Schwimmbädern ermöglicht. Ferner eignet sich dieses Triazin als Herbizid.

Die nachfolgenden Beispiele erläutern die Erfindung näher.

Beispiel 1

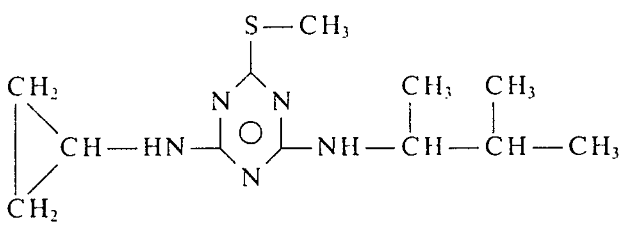

15,1 g 2-Methylthio-4-cyclopropylamino-6-chloro-s-triazin werden in 150 ml Toluol gelöst und bei Raumtemperatur 6,1 g $\alpha,\beta$-Dimethylpropylamin zugetropft. Nach 1 Stunde Rühren 9,2 g 30,4%ige Natronlauge zugegeben und die Mischung 2 Stunden auf 45–50°C erwärmt. Das Toluol wird abgetrennt und der Rückstand neutral gewaschen und getrocknet. Das erhaltenen 2-Methylthio-4-cyclopropyl-amino-6-($\alpha,\beta$-dimethylpropylamino)-s-triazin wird destilliert. (Kp. 146–147°/0,001 mm).

Beispiel 2

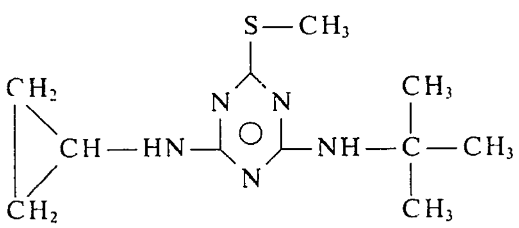

10,4 g 2-Methylthio-4-t.butylamino-6-chloro-s-triazin werden in 40 ml Toluol gelöst und bei 22–32°C 2,57 g Cyclopropylamin zugestopft. Nach 1 Stunde Rühren werden 5,92 g 30%ige Natronlauge zugegeben und während 2 Stunden auf 35°C erwärmt. Das Toluol wird abgetrennt und der Rückstand neutral gewaschen und getrocknet. Das erhaltene 2-Methylthio-4-t.butylamino-6-cyclopropylamino-s-triazin wird aus Methanol/Wasser umkristallisiert (Fp. 121–123°).

## Beispiel 3

2-Methylthio-4-äthylamino-6-(α,β-dimethylpropylamino)-s-triazin (Kp. 143–145°/0,001 mm) kann analog hergestellt werden.

## Beispiel 4

Die Untersuchung der Wirkung gegen die beim Meerwasser-Fouling wichtigste Grünlage Enteromorpha erfolgt in steril filtriertem Meerwasser, welches eine Erd-Schreiber-Lösung enthält. Diese Lösung setzt sich aus einem Nährstoffextrakt, Phosphat und Nitrat zusammen. Die Bebrütung von Enteromorpha intestinalis findet im Lichtthermostaten bei 18°C unter einem 14 h-Licht-10 h-Dunkel-Wechsel statt.

Die auf diese Weise gezüchteten Algen werden den zu untersuchenden Algiziden für kurze Zeit (2 bzw. 4 h) in Meerwasser ausgesetzt. Die minimale Abtötungskonzentration (MKC) wird dadurch bestimmt, daß man die Algen, nach der Einwirkungszeit des eine bestimmte Menge Algizid enthaltenden Meerwassers, diesem entnimmt, sie wäscht, und nach 6–8 wöchiger erneuter Bebrütung in frischem Meerwasser auf Wuchs bzw. Abtötung untersucht.

Die minimale Abtötungskonzentration (MKC) gibt die Stoffmenge an, die notwendig ist, die Alge innerhalb einer bestimmten Zeit so zu schädigen, daß sie sich in frischem Meerwasser nicht mehr erholen kann und abstirbt.

Die Resultate dieser Untersuchungen nach 4 h sind in Tabelle 1, nach 2 h in Tabelle 2 zusammengestellt. Die außerordentlich tiefen MKC-Werte der Triazine I, II und III zeigen die überraschend rasche Wirksamkeit dieser Verbindungen.

Tabelle 1

| Algizid | MKC in ppm nach 4 h |
|---|---|
| 2-Methylthio-4-äthylamino-6-isopropylamino-s-triazin (gemäß US Pat. Nr. 4 012 503) | 5 |
| 2-Methylthio-4,6-diäthylamino-s-triazin (gemäß JP-A-Nr. 49 061-336) | 5 |
| 2-Methylthio-4,6-diisopropylamino-s-triazin (gemäß JP-A-Nr. 52 021-331) | >5 |
| 2-Methylthio-4-cyclopropylamino-6-isopropylamino-s-triazin | >5 |
| 2-Methylthio-4-cyclopropylamino-6-(1',3'-dimethylbutylamino)-s-triazin | 5 |
| 2-Methylthio-4-cyclopropylamino-6-sec.butylamino-s-triazin | >5 |
| 2-Methylthio-4-n-propylamino-6-t.butylamino-s-triazin | >5 |
| 2-Methylthio-4-äthylamino-6-t.butylamino-s-triazin | 5 |
| 2-Methylthio-4-cyclopropylamino-6-(α,β-dimethylpropylamino)-s-triazin (I) | 0,5–1 |
| 2-Methylthio-4-cyclopropylamino-6-6-t.butylamino-s-triazin (II) | 0,5 |
| 2-Methylthio-4-äthylamino-6-(α,β-dimethylpropylamino)-s-triazin (III) | 1 |

Tabelle 2

| Algizid | MKC in ppm nach 2 h |
|---|---|
| 2-Methylthio-4-äthylamino-6-isopropylamino-s-triazin (gemäß US Pat. Nr. 4 012 503) | >7 |
| 2-Methylthio-4,6-diäthylamino-s-triazin (gemäß JP-A-Nr. 49 061-336) | >7 |
| 2-Methyltio-4-cyclopropylamino-6-(1',3'-dimethylbutylamino)-s-triazin | >7 |
| 2-Methylthio-4-äthylamino-6-t.butylamino)-s-triazin | >7 |
| 2-Methylthio-4-cyclopropylamino-6-($\alpha,\beta$-dimethylpropylamino)-s-triazin (I) | 1–2 |
| 2-Methylthio-4-cyclopropylamino-6-t.butylamino-s-triazin (II) | 1–2 |
| 2-Methylthio-4-äthylamino-6-($\alpha,\beta$-dimethylpropylamino)-s-triazin (III) | 2–3 |

**Patentansprüche**

1. Verwendung eines Triazins der Gruppe bestehend aus 2-Methylthio-4-cyclopropylamino-6-($\alpha,\beta$-dimethylpropylamino)-s-triazin (I), 2-Methylthio-4-cyclopropylamino-6-t.butylamino-s-triazin (II) und 2-Methylthio-4-äthylamino-6-($\alpha,\beta$-dimethylpropylamino)-s-triazin (III) als Algizid gegen Meeresalgen.
2. Verwendung der Triazine I, II und III gemäß Anspruch 1 als Algizide gegen Enteromorpha.
3. Verwendung der Triazine gemäß Anspruch 1 zum Schützen von Materialien gegen Meeresalgenbewuchs.
4. Verwendung gemäß Anspruch 3, dadurch gekennzeichnet, daß die Triazine in einem Schutzanstrichmittel verwendet werden.
5. Verwendung gemäß Anspruch 4, dadurch gekennzeichnet, daß das Schutzanstrichmittel eine Antifoulingfarbe ist.
6. Verwendung der Triazine gemäß Anspruch 1 zum Schützen von Schiffskörpern, Bojen, Wasserbauten und Fischernetzen.
7. Verwendung der Triazine gemäß Anspruch 1 zum Schützen von Meerwasserkühlsystemen.
8. 2-Methylthio-4-cyclopropylamino-6-($\alpha,\beta$-dimethylpropylamino)-s-triazin (I).

**Claims**

1. Use of a triazine of the group comprising 2-methylthio-4-cyclopropylamino-6-($\alpha,\beta$-dimethylpropylamino)-s-triazine (I), 2-methylthio-4-cyclopropylamino-6-tert-butylamino-s-triazine (II), and 2-methylthio-4-ethylamino-6-($\alpha,\beta$-dimethylpropylamino)-s-triazine (III) as an algicide to combat sea-water algae.
2. Use of the triazines I, II and III according to Claim 1 as algicides to combat Enteromorpha.
3. Use of the triazines according to Claim 1 for the protection of materials from becoming overgrown with sea-water algae.
4. Use according to Claim 3, characterised in that the triazines are applied in a protective coating composition.
5. Use according to Claim 4, characterised in that the protective coating composition is an antifouling paint.
6. Use of the triazines according to Claim 1 for the protection of ships' hulls, buoys, structures in water, an fishing nets.
7. Use of the triazines according to Claim 1 for the protection of sea-water cooling systems.
8. 2-Methylthio-4-cyclopropylamino-6-($\alpha,\beta$-dimethylpropylamino)-s-triazine (I).

**Revendications**

1. Utilisation d'une triazine du groupe de la 2-méthylthio-4-cyclopropylamino-6-($\alpha,\beta$-diméthylpropylamino)-s-triazine (I), de la 2-méthylthio-4-cyclopropylamino-6-t-butyl-amino-s-triazine (II) et de la 2-méthylthio-4-éthylamino-6-($\alpha,\beta$-diméthylpropylamino)-s-triazine (III) comme algicide contre des

algues marines.

2. Utilisation des triazines I, II et III selon la revendication 1 comme algicides contre Enteromorpha.

3. Utilisation des triazines selon la revendication 1 pour protéger des matériaux contre la propagation et le recouvrement d'algues marines.

4. Utilisation selon la revendication 3, caractérisée en ce que les triazines sont employées dans un produit de revêtement protecteur;

5. Utilisation selon la revendication 4, caractérisée en ce que le produit de revêtement protecteur est une peinture antifouling, c'est-à-dire contre les encrassements.

6. Utilisation des triazines selon la revendication 1 pour protéger des coques de navires, balises, installations marines et filets de pêche.

7. Utilisation des triazines selon la revendication 1 pour protéger des systèmes de refroidissement à l'eau de mer.

8. A titre de produit industriel nouveau, la 2-méthylthio-4-cyclopropylamino-6-($\alpha$,$\beta$-diméthylpropylamino)-s-triazine (I).